(19) Europäisches Patentamt / European Patent Office / Office européen des brevets

(11) **EP 4 534 986 B1**

(12) **EUROPEAN PATENT SPECIFICATION**

(45) Date of publication and mention
of the grant of the patent:
**25.03.2026 Bulletin 2026/13**

(21) Application number: **23214856.9**

(22) Date of filing: **07.12.2023**

(51) International Patent Classification (IPC):
*G01N 21/95* (2006.01)      *B65G 43/00* (2006.01)
*G01B 11/02* (2006.01)      *G01B 11/04* (2006.01)
*G01B 11/245* (2006.01)     *G01B 21/06* (2006.01)
*G01B 21/20* (2006.01)      *G01B 7/02* (2006.01)

(52) Cooperative Patent Classification (CPC):
**G01B 21/06; B65G 43/02; G01B 7/023; G01B 21/20**

(54) **DEVICE AND METHOD FOR MEASURING THE DEFECTS OF RUBBER CARCASS ON THE EDGES OF A CONVEYOR BELT RUNNING ON ROLLERS**

VORRICHTUNG UND VERFAHREN ZUR MESSUNG VON DEFEKTEN EINER GUMMIKARKASSE IN RANDBEREICHEN EINES AUF ROLLEN LAUFENDEN FÖRDERBANDES

DISPOSITIF ET MÉTHODE POUR MESURER LES DÉFAUTS DE LA CARCASSE EN CAOUTCHOUC SUR LES BORDS D'UNE BANDE TRANSPORTEUSE CIRCULANT SUR DES ROULEAUX

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **05.10.2023 PL 44630823**

(43) Date of publication of application:
**09.04.2025 Bulletin 2025/15**

(73) Proprietor: **Akademia Gorniczo-Hutnicza im. Stanislawa**
**Staszica w Krakowie**
**30-059 Krakow (PL)**

(72) Inventors:
• **Kwasniewski, Jerzy**
  **30-211 Kraków (PL)**
• **Molski, Szymon**
  **31-901 Kraków (PL)**

(74) Representative: **Rogozinska, Alicja**
**Polservice**
**Kancelaria Rzecznikow**
**Patentowych sp. z o.o.**
**Bobrowiecka 8**
**00-728 Warszawa (PL)**

(56) References cited:
**AU-A1- 2012 216 769      US-A1- 2010 294 624**
**US-A1- 2023 091 198**

**Description**

The subject of the invention is a device and method for measuring rubber matrix defects, e.g. losses, on the edges of a belt of belt conveyors equipped with steel cords and running on rollers. The invention makes it possible to identify the presence of operational rubber defects on edges of a rubber conveyor belt, regardless of its location on the rollers and transverse displacement of said belt with respect to a measuring system.

Conveyor belts undergo wear during operation as a result of longitudinal loads, abrasive and impact effects of the transported feed material, influence of atmospheric conditions, aging of rubber, etc. Damage to belt edges occurring during operation can be quickly and easily repaired by local vulcanization or bonding. Damage or defects to the belt edges, detected at the initial stage, allows to perform their ongoing vulcanization repairs and eliminate the likelihood of rapid damage buildup, loss of transported material and increase the safety of belt operation.

Damage to the belt, especially its abrasive wear, is expressed by a change in a thickness dimension and thanks to continuous measurement of a thickness during operation it is possible to perform ongoing repairs that eliminate the rapid buildup of such damage.

Devices for continuous measurement of the thickness of a conveyor belt are known, for example, from the Polish utility model description No. Ru 50363, or from the European invention description No. EP 1811266, that solutions respectively have a distance sensor cooperating with a measuring roller running directly on a surface of a conveyor belt in its lower or return course, said surface is supported on the other side by the base surface of the roller or a thrust sliding element. The measuring roller is seated in a bearing in the frame of the device on a rocker. The arm of the rocker is pivoted perpendicularly to the surface of the belt and acts on a measuring instrument connected to an electronic thickness analyzer and recorder of results.

Also known is a solution entitled: "Device for non-contact measurement of a condition of a conveyor belt cover" ("Urządzenie do bezkontaktowego pomiaru stanu ostony taśmy przenośnikowej") protected by patent PL 236716, the subject of which is a device for non-contact measurement of a condition of a conveyor belt cover, applicable for checking the technical condition of a surface of conveyor belts, such as rubber or textile belts. This device, for non-contact measurement of the condition of a conveyor belt cover containing a structural frame on which at least one series of movable nozzles is located, which nozzles are mechanically engaged to displacement sensors and connected to a pneumatic system supplied from a source of compressed medium. The device is distinguished by the fact that each nozzle is attached to one end of a lever which is pivotally connected to the structural frame in such a way that its free rotation in a plane parallel to a direction of measurement and in a plane perpendicular to a surface of the conveyor belt under examination is ensured while the other end of the lever is mechanically engaged to a displacement sensor which is connected wirelessly or by means of a signal cable to an electronic signal conditioning system, which is connected by means of a signal cable or wirelessly to an inverter of a drive system of a conveyor drive drum. Furthermore, a connection of each nozzle to a pneumatic system is realized by means of flexible hydraulic hoses.

In addition, methods are known for monitoring the shape or damage of a band element moving relative to a measuring head containing pneumatic transducers. Operation of said transducer is based on a change in parameters of an air flowing through its elements, which change is caused by an increase or decrease in a measuring gap between a face of a nozzle and a surface of tested band. For example, from Japanese patent description JP20002277384A a device is known for detecting defects of a band which can be, for example, a sheet of metal, and irregularities in its surface by means of a pneumatic transducer located on a stand and connected to a source of compressed air through a pressure regulator.

A system that is known from US patent description US4031741A allows continuous monitoring of the flatness of a band across its entire width applicable to rolling mills, paper factories and the like. This system includes a measuring head in the form of a frame with a width corresponding to the width of the band to be monitored and set in a direction parallel to its width. In said measuring head pneumatic measuring elements that are spaced at fixed intervals and connected to a source of compressed air through a pressure regulator. Each of the pneumatic measuring elements has a transducer for changing a pressure to electrical quantities, which is connected via a signal modulator to a device for displaying the measured quantities.

From international patent application WO2009061731A1 a solution is known for modifying a mechanical measuring device by replacing a series of measuring rollers embedded in a frame with pneumatic heads that do not contact a band of material under test. The device includes a structural frame on which a series of movable nozzles is located and connected to a pneumatic system supplied by a source of compressed medium. Each pneumatic head is movable along a single axis perpendicular to the surface to be tested. A distance of this displacement is measured by a linear displacement sensor to which it is coupled.

Also known is a solution according to Patent No. PL 215143 titled: "Device for operational monitoring of conveyor belt thickness, especially in long transport lines of the mining industry" ("Urządzenie do eksploatacyjnego monitorowania grubości taśmy przenośnika, zwlaszcza w długich magistralach transportowych przemysłu wydobywczego"), used in output transport equipment in the mining industry. This device can be used for measuring a thickness of fabric-rubber belts and rubber belts reinforced with steel cords or nets. The device according to this invention has, as in the above-described solutions, a frame on which distance sensors are mounted and arranged perpendicularly to a surface of the belt in its lower course, which sensors producing signals that are transmitted continuously to an electronic thickness analyzer and the results from the thickness analyzer are recorded in a recorder. The solution is distinguished in that the frame of the device is "C" shaped having parallel horizontal arms with a length of not less than a half of a width of a conveyor belt. Two non-contact distance sensors are mounted coaxially on the arms, spaced at a base distance and facing each other. Such a solution enables non-contact differential thickness measurement, which eliminates technical problems associated with belt thickening at overlapping joints and makes a measured results independent on transverse belt vibrations. According to this solution, a single distance sensor located just above the belt under test is used, which sensor is paired with a second sensor located under the belt. A signal from the differential system is recorded regardless of the vibrations of the belt running on rollers, which signal informs about a thickness of the belt. The sensor system is movable across the belt to determine its thickness, selectively across its width.

Such a system of two sensors cannot be used for measuring defects in edge areas of a conveyor belt due to instability of the belt edges during its operation (i.e. vibrations) and lateral movements of the belt over a wide range of up to from 10 to 20 centimeters, as well as due to large defects usually occurring at the belt edges.

Wearing and a tear of conveyor belts occurring during operation are particularly damaging in long conveyors i.e. of long lengths, and particularly critical places in which such damage occur are joints of belt sections, connected one to another by overlapping at their ends by means of vulcanization or gluing. Minor damage occurring during operation - which at their initial stage can be quickly and easily repaired by local vulcanization or gluing can spread rapidly under the dynamics of movement under load, and escalation of damage can lead to such damage that is no longer repairable and to the need to replace the entire belt section and significant production losses. Damage to the belt, especially its abrasive wear, results in a change in its thickness dimension. By continuous measurement of the belt thickness during operation it is possible to make ongoing repairs that eliminate rapid escalation of said damage.

It is particularly difficult to detect at an early stage conveyor belt damage occurring in its edge areas, especially belt defects or loss at its edges, which can cause adverse effects similar to those described above.

Also known from patent publication No. AU2020203474 is a device and method for detecting transverse displacements, that is a belt wander, of a trough conveyor belt, to the right and to the left, in which the actual position of the belt edges during operation is measured, or detected, by means of a pair of detectors, advantageously ultrasonic probes, which are positioned on the sides of the conveyor belt, at a mutual distance greater than the width of the belt measured between its both edges and covering the belt edge areas, respectively left and right, opposite to each other, and at least one sensor, preferably several sensors, in the form of proximity switches which are arranged near the top surface of the belt, at a certain distance from the surface of its two edge areas, in between. The result of the measurement is used to evaluate the condition of a conveyor belt and the need of repair of it by means of an analysis unit on the basis of the results of measuring lateral displacements of the belt beyond a designated threshold value. Wherein if the right and the left belt edge displacements are equal, the result is taken into account in the evaluation. On the other hand, if the measured or detected displacements are not equal in both directions, they are not taken into account in order to avoid the influence of losses or defects occurring in the belt edges in such a case on the result of the measurement. The described solution is not suitable for use in conveyors that operate under conditions of large displacements of the edge areas of the conveyor belt both in the width direction, i.e. transverse to the conveying direction, and in the vertical direction (vibration), perpendicular to the conveying direction. Moreover, the above solution does not allow determining a size and location of losses and defects in the belt edge areas.

Also known are a device and method of measuring the thickness of one cover of a steel cord reinforced conveyor belt whilst the belt is moving as described in AU 2012 216 769 A1 and a device and method for detecting a rip or tear in a conveyor belt as described in US 2023/091198 A1.

The purpose of the invention is to develop a device that allows the rapid identification of worn areas of the rubber carcass or matrix in edge areas or periphery areas of a belt of the conveyor belt equipped with steel cords forming a steel core of the belt, and thus allowing the rapid repair of these worn areas. The purpose of the invention is also to provide a device and a method for determining the location of such defects.

The purpose of the invention is realized by a device for measuring defects and losses of the rubber carcass on edges of a belt equipped with steel cords for conveyor belts running on rollers, whereby the device makes it possible to monitor, measure, record and analyze of a condition of a geometry of belt edge areas. In addition, the device includes a measuring system that is primarily characterized by efficiency and high measuring accuracy, regardless of transverse, vertical and longitudinal vibrations of the belt, as well as high environmet dustiness, that is under conditions in which other devices such as cameras cannot be used. The device according to the invention provides safety as well as simple operation.

## Essence of the invention

According to the invention, a device according to claim 1 for measuring defects in edge areas of a conveyor belt, especially losses of a rubber carcass of belt edges of conveyor belts equipped with steel cords forming a steel core of said belt running in a circulating motion on rollers, is proposed. The device according to the invention includes a support structure located on both lateral sides of the conveyor belt, on which, perpendicularly to a direction of movement of the conveyor belt, in the lower or return run of the conveyor, distance sensors are located, producing signals which are continuously transmitted to an electronic analyzer connected to said distance sensors by way of signal transmitting connection and recorded, wherein the analyzer is connected to an industrial computer and wherein the support structure of the device includes two brackets located next to the conveyor belt, opposite to each other on both lateral sides of this conveyor belt, respectively. On each of the brackets distance sensors are arranged respectively opposite with respect to each other and coaxially to each other, spaced by a base spacing and facing each other, which distance sensors operate in a differential system allowing non-contact distance measurement by way of the differential method.

The essence of the invention is in that a measuring strip is attached to each of the brackets of the support structure, forming a measuring system consisting of two measuring strips having distance sensors attached to each of these measuring strips and arranged opposite to each other, which are differentially connected to the analyzer allowing measurement of a width h1 of the conveyor belt, wherein each measuring strip contains at least 3 distance sensors, and preferably more distance sensors, which match in pairs and a spaced on opposite strips as mutually paired with each other respectively on both sides of the conveyor belt and located at a mutual distance h2 from each other. The distance h2 is greater than the distance h1. In addition, bars-observers 10 in the form of magnetometer strips 10 each equipped with at least one magnetometer sensor or plurality of magnetometer sensors are mounted on each bracket of the support structure, wherein the magnetometer sensors being connected by signal transmitting connection to the analyzer.

Preferably, the measuring strip equipped with distance sensors is located at a distance H1 from the geometric center of the cross-section of the nearest outermost edge steel cord of the conveyor belt and at a distance x1 from the nearest edge of the conveyor belt, while the distance between the edge of the conveyor belt and the geometric center of the outermost edge steel cord located closest to it is $b_{nom}$, wherein the distance H1 being equal to the sum of the distances $x1 + b_{nom}$ for defect-free on its edges conveyor belt having a nominal width h1, and wherein the distance H1 being less than the sum of the distances $x1 + b_{nom}$ at the point where the defect in the form of a loss occurs in the edge area of the conveyor belt.

A further aspect of the invention proposes a method according to claim 10 for measuring defects in the form of losses in edge areas of a conveyor belt, especially defects of a rubber carcass of the belt edges of a conveyor belt equipped with steel cords forming a steel core of the belt by detecting and determining the location of a defect in the edges of the conveyor belt, wherein computer analysis is carried out using an analyzer and an industrial computer of a device for measuring defects in edge areas of a conveyor belt, which the analyzer and the computer are connected by way of signal communication connection to distance sensors located on both lateral sides of the conveyor belt, opposite to each other. The essence of the invention is in that a computer analysis of signals from a number of distance sensors is carried out which sensors are arranged in a paired differential arrangement with each other and matched in pairs on measuring strips, which are built on both lateral sides of the conveyor belt, wherein signals from magnetometer sensors arranged on magnetometer strips, which are arranged horizontally, transversely over a surface of the conveyor belt over its edge areas. Wherein the distances H1, x1 and $b_{nom}$ are determined and it is checked whether the inequality 1) **H1 - x1 < $b_{nom}$** is met, where H1 - is the distance between the measuring strip with distance sensors and the geometric center of the cross-section of the outermost edge steel cord, located closest to the given belt edge; x1 - is the distance of the measuring strip with sensors from the edge of the conveyor belt, and $b_{oom}$ - is the nominal width of the belt edge, where if the above inequality is met, it is considered that a defect in the edge of the conveyor belt at the given location has been detected.

Advantages of the solution according to the invention lie in the use of two non-contact measuring strips that ensure measurement in a differential arrangement, thus ensuring that the measuring system is insensitive to transverse vibrations of the conveyor belt at the time of measuring. In addition, the device according to the invention shows insensitivity to displacements of the conveyor belt along the width of the rollers. The solution according to the invention uses a matched in pairs that is paired set of distance sensors arranged on measuring strips, for example, laser distance sensors, ultrasonic or similar, covering a field of vision of the belt of a given thickness and its surroundings. In this way possibility of observing the belt edges, taking into account possible significant vibrations of the belt is guaranteed, which leads to insensitivity of the measurement of defects to possible lateral displacements of the belt and vibrations even in the case of large defects or losses in its edge areas. This favorable effect according to the invention is achieved in particular due to the corresponding field of view of the measurement sensor unit.

In addition, the position of defect locations in the edges of the conveyor belt is determined by using magnetometer sensors arranged on bars-observers, hereinafter referred to as magnetometer strips, located horizontally with respect to the belt surface, above the belt surface. The device according to the invention can be used in operating areas exposed to high dust and with limited permeability to a light radiation, including in the infrared and ultraviolet range.

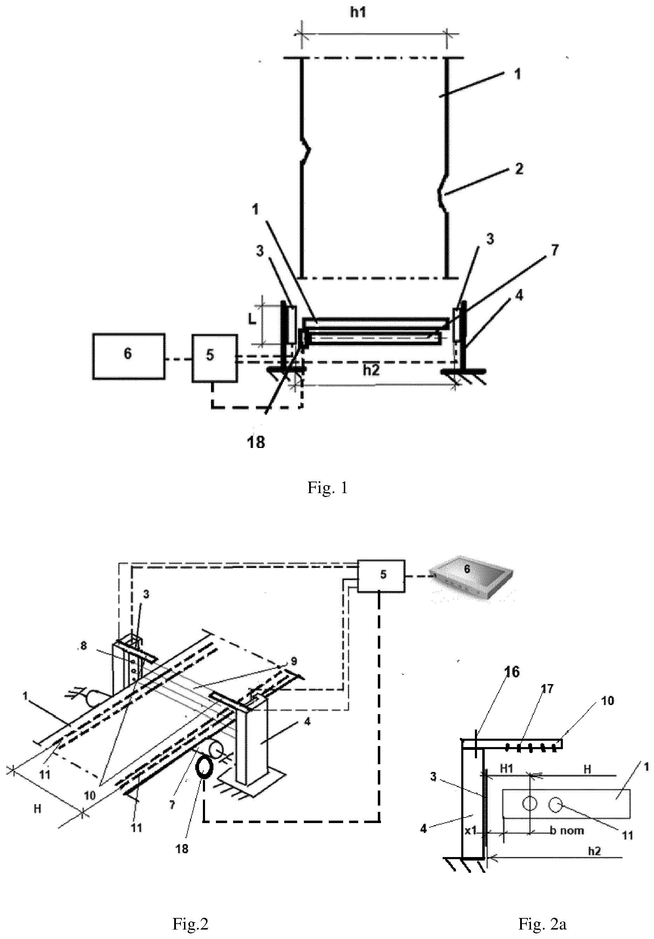

The device according to the invention in the embodiment is shown in the drawing, in which fig. 1 schematically presents the device according to the embodiment of the invention in a transverse view with respect to the conveyor belt run, fig. 2 - the device in the embodiment of the invention in schematic perspective view, and fig. 2a - a schematic cross-sectional view of a section of the device from one side of the belt.

A device for measuring defects of the rubber carcass on belt edges of a conveyor belt equipped with steel cords forming a steel core of the belt, which runs along the rollers in the embodiment of the invention consists of two measuring strips 3, each of which is provided with a certain number of distance sensors 8 distributed in spaces along it, the number amounts to at least 3 or more, preferably from 3 to 10 sensors, in particular more than 10 sensors 8, preferably the same number, e.g. laser sensors, ultrasonic sensors, etc. placed in a differential system or arrangement, wherein the measuring strips 3 are located and fixed on the lower or return run of the conveyor belt on an independent self-contained support structure 4. The support structure consists of two opposite brackets 4 placed on both lateral sides of the conveyor belt 1, opposite to each other, with additional, at least two, bars-observers 10, hereinafter referred to as magnetometer strips 10, equipped with magnetometer sensors 17 and fixed, preferably one, on each bracket of the support structure 4 in such a way as to extend horizontally in the direction to the surface of the belt 1, above this surface of the conveyor belt 1 in the direction perpendicular to the transfer direction of conveyor. Further the device consists of a measuring and analyzing module 5, hereinafter referred to as analyzer 5, with which both the distance sensors 8 and the magnetometer sensors 17 are connected by signal transmitting connection, as well as an industrial computer 6 and possibly a display element (not shown) connected to the analyzer 5.

The conveyor belt 1 having nominal width h1 has, due to operation, defects and losses of rubber carcass or matrix in its edge areas 2 along the length of the belt 1, which runs on rollers 7. Measuring strips 3 with distance sensors 8 are arranged opposite to each other on both lateral sides of the conveyor belt 1 at a distance h2 from each other, which distance is greater than the width h1 of the belt. This positioning of them allows the conveyor belt 1 to move freely laterally on the rollers 7. Thus. it is not possible for the belt 1 to damage the distance sensor measuring arrangement 8 arranged on the measuring strips 3, which are matched in pairs and paired with each other, i.e. located and cooperating in pairs one to another, so as to determine the field of view 9 of the sensors between each other. The use of a measuring arrangement of distance sensors 8 mounted on the measuring strip 3 and operating on a differential principle makes it possible to become independent of the relative position of the conveyor belt 1 on the rollers 7 at the measuring point.

In addition, the device is equipped with magnetometer bars 10 (bars-observers 10) with magnetometer sensors 17 arranged on them and affixed thereto, wherein there is at least one magnetometer sensor 17 or more, advantageously several magnetometer sensors 17, on each magnetometer strip 10 (bar-observer), which makes it possible to determine the width H of the steel core, between the geometric centers of the outermost edge steel cords 11, consisting of the steel cords 11 of the conveyor belt 1, as well as to determine the distance H1 between the measuring strip 3 having distance sensors 8 distributed on it and the geometric center of the cross-section of the outermost edge steel cord 11 located closest to the given belt edge, whereby the nominal distance of the outermost edge steel cord 11, or more precisely the center of its cross-section from the edge of the conveyor belt 1 is defined as $b_{nom}$, wherein the distance of the measuring strip 3 having distance sensors 8 from the edge of the belt 1 is x1. The signals from the distance sensors 8 on the measuring strips 3 and from the magnetometer sensors 17 on the magnetometer strips 10 are transmitted to the analyzer 5 in any known way and by any known means, either wired or wireless, wherein an arrangement of two measuring strips 3 having distance sensors 8 differentially connected by way of differential system to the analyzer A is used, that allows measuring of the width h1 of the belt 1 and recording in the industrial computer 6.

In the configuration of the device according to the invention, the distance H1 is equal to the sum of the distances $x1 + b_{nom}$ for the defect-free edges of the conveyor belt 1 of nominal width h1, and the distance H1 is less than the sum of the distances $x1 + b_{nom}$ at the place in which the defect or loss in the edge area of the conveyor belt 1 occurs.

In addition, the device includes an encoder 18, which is attached to the structural elements of the attachment of the rollers 7 of the conveyor belt, in the vicinity of the roller 7 and also one of the measuring strips 3. The encoder being connected by signal transmitting connection to the analyzer 5. The signal from the encoder 18 is transmitted to the analyzer 5 and makes it possible to determine the location of the defect.

It is important to use an appropriate length of measuring strips 3 with distance sensors 8 and properly calibrated software of the analyzer 5 so as to ensure the correctness of the measurement during possible transverse vibrations of the tested conveyor belt 1 during operation.

The measuring strips 3, depending on the dimensions of the distance sensors 8 used, contain from 3 to 20 distance measuring sensors 8, arranged one behind the other in an identical manner in each of the two measuring strips 3 and in a pair-matched manner to each other.

The length of the measuring strips L is selected according to the number of distance sensors 8 and the maximum possible vibration of the belt 1 edges, which can occur during operation, amounting up to 10 to 20 centimeters.

Calibration of the measuring system and the analyzer 5 software consists in setting of the registration parameters for the conveyor belt 1 of nominal width h1, i.e. without defects in its width, and in parallel positioning of the field of view 9 of the distance sensors 8.

The distance sensors 8 arranged in the measuring strips 3 and the magnetometer strips 10 provided with magnetometer sensors 17 arranged in them are equipped with their own internal power supply.

In another aspect of the invention, the location of a defect 2, in particular a loss 2, in the edge area of the conveyor belt 1 is determined and detected by means of the above-described device according to the invention by method of measuring defects 2 in the edge areas of a conveyor belt 1, especially defects (losses) of the rubber carcass of belt edges of the conveyor belts equipped with steel cords forming a steel core of the belt, by means of the computer analysis using an analyzer 5 and an industrial computer 6, on the basis of signals from distance sensors 8 arranged on measuring strips 3 and magnetometer sensors 17 arranged on magnetometer strips 10.

By means of computer analysis using industrial computer 6 on the basis of the obtained results of measurements of the above-mentioned and explained distances H1, x1 and $b_{nom}$, the locations of defects 2 or losses 2 in the belt edges and the size of each defect are identified. The identification and detection of defect location is realized by analyzing the following inequality:

$$1) \quad H1 - x1 < b_{nom}$$

wherein a defect is detected if this inequality is met or satisfied,
where:

$b_{nom}$ - is the nominal width of the belt edge, i.e. the distance of the conveyor belt 1 edge from the geometric center of the cross-section of the extreme edge steel cord 11 of the steel core, located closest to each conveyor belt edge;

H1 - means the distance between the measuring strip 3 with distance sensors 8 and the geometric center of the cross-section of the extreme edge steel cord 11 of the respective side of the conveyor belt 1, located closest to the belt 1 edge; and

x1 - is the distance of the measuring strip 3 with distance sensors 8 from the respective closest edge of the conveyor belt 1;

Fulfillment of the above-described relationship indicates the occurrence of a defect (loss) in the conveyor belt 1.

The method for determining the occurrence of the defect in the edges of the conveyor belt 1 consists in determining, by means of a device for measuring the defects of the rubber carcass in the belt edges of conveyor belts according to the invention, on the basis of computer analysis, by employing an analyzer 5 and an industrial computer 6, of the signals from the distance sensors 8 and from magnetometer sensors 17 the distances H1, x1 and $b_{nom}$ according to the definitions described above and checking whether the inequality H1 - x1 < $b_{nom}$ is satisfied.

The distance sensors are arranged in a paired configuration with each other in differential system on the both measuring strips 3, which are built on both lateral sides of the conveyor belt 1. The magnetometer sensors 17 are arranged on magnetometer strips 10 located as projecting horizontally above the surface of the conveyor belt 1, at least on its edge sections.

If this inequality is satisfied, it is considered that a defect has been detected in the edge of the conveyor belt 1 at a given location. The location of the defect is determined on the basis of the signal obtained from the encoder 18 as described earlier.

Examples of magnetometer sensors 17 used in the magnetometer strips 10, so-called bar-observer 10, include but are not limited to magnetometer sensors of the XEN 1210 or XEN 1220 type with a converter interface. On the other hand, the measuring bar 3 may use CMOS Series LR-X digital laser sensors or HC-SR04 ultrasonic distance sensors as distance sensors 8, for example, but other suitable types of sensors can be used.

In order to achieve the technical effect of being able to measure, under harsh and dusty operating conditions, the defects in the edge areas of the conveyor belt 1, a measuring system consisting of a large number or a plurality of distance sensors 8, arranged on measuring bars 3, which sensors are paired with each other, advantageously, from 10 to 20 distance sensors 8 or more may be arranged on each strip 3, working in a differential arrangement. These distance sensors 8 are arranged along the height of the measuring strips, along a length that allows the measurement covering the entire thickness of the cross-sectional area of the conveyor belt 1 together with sections that include sections of the amplitude of vibrations occurring and/or movements in transvers and perpendicular directions to the direction of movement of the belt 1 during operation. Usually this length covering the entire range of possible movements of the conveyor belt 1, and more precisely of its edges, in the form of vibrations occurring during the transfer of a load in a vertical or diagonal directions with a vertical component. The distance sensors 8 are arranged on the measuring strip 3 in mutually obtainable close proximity to each other taking into account dimensional and technical considerations, which significantly increases the field of view 9 of the entire measuring system of the sensors 8, which is marked in fig. 2. This arrangement of the distance sensors 8 makes the measurement independent on the transverse vibrations of the edges of the conveyor belt 1, as well as of the entire belt 1. The above-described distance sensors pairing that is from 10 to 20 distance sensors 8 operating in a differential arrangement in close proximity to each other significantly increases the field of view 9 of the measurement system and causes the measurement process independent of significant vibrations and transverse movements of the conveyor belt 1, particularly its edges, to the extent of 10 to 20 centimeters. The measurement can be performed independently on these movements and vibrations. By means of such a measuring system, it is possible to unambiguously determine the size of defects 2 in the belt edge areas.

The both measuring strips 3 are positioned in such a way that each pair of distance sensors 8 arranged on them respectively is paired with each other (arranged in mutually matching positions on opposite strips) in the so-called field of view 9 of the sensors 8 (marked in fig. 2). Due to the different sizes of the measuring elements, i.e. the distance sensors 8, the sensors are arranged in the measuring strips 3 with the possibility of mutual displacement, i.e. they can be moved with respect to each other and relative to a certain reference point, advantageously relative to the support structure 4, which in this case constitutes the structural base 4.

The position of the movable measuring strips 3, i.e. fixed movably on the brackets of the support structure 4, prior to measurement is each time aligned and correlated one to another taking into account the unevenness of the environment on which the support structure of the diagnostic system rests, so as to provide a field of view 9 of the sensors. Signals from the measuring i.e. distance sensors 8 are sent to a structurally independent measuring and analyzing module 5 which is a separate box in an IP65 waterproof housing, connected to an industrial computer 6.

The correctness of the measurement during possible transverse vibrations of the tested belt 1 during its operation is achieved by using the appropriate length L of the measuring strips 3, as explained above, with distance sensors 8, and by properly calibrated measuring system and software of the analyzer 5 and the cooperating industrial computer 6 (as described above).

The lower run of the conveyor belt 1 having the width h1, on which the measuring device according to the invention, constituting the diagnostic system, is mounted, can move transversely on the rollers 7 during operation under the influence of, for example, the load of excavated material, within the limits of plus or minus 20 cm. Thus, the mutual distance h2 of the measuring strips 3 from each other must be selected according to the relation: 2) $h2 = h1 + 40$ cm.

Where: h1 is the nominal width of the conveyor belt 1, and h2 is the distance between the measuring strips 3, located opposite each other on both sides of the conveyor belt 1 and equipped with distance sensors 8 whose position relative to each other is correlated so that the distance sensors 8 arranged on each measuring strip 3 are located opposite one another as matching in pairs that is mutually paired with each other in their field of view 9.

Any defect 2 in the width of the belt, or loss or pulling out of the belt edge, which is different from the nominal dimension is recorded and signaled, for example, on the industrial computer screen. At a constant speed of movement of the conveyor belt, identification of the defect 2 location in the belt 1 is made in order to repair the belt.

In an embodiment of the invention, an encoder 18 is used for identification of the defect 2 location, which encoder is connected by signal communication to an analyzer 5 and attached to available nearby structural elements holding the roller 7, in the immediate vicinity of the respective roller 7.

During computer analysis, the locations of defects can be identified and their size can be assessed, wherein the following quantities being determined:

h1- denotes the measured width of the conveyor belt 1;

h2- denotes the distance between the measuring strips 3 with arranged on it distance sensors 8;

H1- denotes the distance between the measuring strip 3 with distance sensors 8 and the geometric center of the cross-section of the outermost steel cord 11 located closest to the given belt 1 edge;

H - means the width of the steel core that covers all steel cords 11 arranged in the conveyor belt 1, including the outermost edge steel cords 11closest to the edges of the belt 1, between the geometric centers of both outermost edge steel cords 11, left and right; the width of H should be less than the width h1 and the distance h2;

x1 - means the distance of the measuring bar 3 with sensors 8 from the edge of the conveyor belt 1;

$b_{nom}$ - means the nominal distance of the edge of the conveyor belt 1 from the geometric center of the cross-section of the outermost edge steel cord 11 located closest to the edge of the given conveyor belt 1;

In an embodiment of the invention, the magnetometer strips 10, so-called bars-observers 10, are attached to the support structure 4, for example, screwed to said support structure 4 with a screw 16, and are matched in pairs i.e. paired with each other, that is, on each bracket of support structure 4 one magnetometer strip 10 is located, respectively, protruding from the bracket in the transverse direction of the conveyor belt 1 above the surface of its edge area. Whereby, the magnetometer strips 10 are located opposite to each other and arranged as horizontally positioned with respect to the surface of the conveyor belt 1 to be tested, and they are arranged in alignment with each other in one cross-section of this belt 1. Such a number of magnetometer sensors 17 are arranged on each bar-observer 10, depending on the supplier, so that it has a length in the horizontal direction allowing observation of the conveyor belt 1 at least in the section from the edge of the given conveyor belt 1 to the outermost edge steel cord 11, and more precisely to the geometric center of the cross-section of that given outermost edge steel cord 1, from given edge side of the conveyor belt 1. The length of the magnetometer bar 10 is at least equal to the sum of the distances $x1 + b_{nom}$, at the expected operational lateral displacements of the belt, but may be greater.

## Claims

1. A device for measuring defects in edge areas of a conveyor belt (1), especially defects in a rubber carcass of belt edges of the conveyor belt (1) which is equipped with steel cords (11) forming a steel core of the belt, running in a circulating motion on rollers (7), which device includes a support structure (4) located on both lateral sides of the conveyor belt (1), on which support structure (4) perpendicular to the direction of movement of the conveyor belt distance sensors (8) are arranged, in the lower run of the conveyor, the distance sensors (8) produce signals which are transmitted continuously to an electronic analyzer (5) connected to them by way of signal transmitting connection and recorded, wherein the analyzer (5) being connected to an industrial computer (6), wherein the support structure (4) of the device includes two brackets located next to the conveyor belt (1) and opposite to each other on both lateral sides of this conveyor belt (1), on each of which the distance sensors (8) are arranged opposite and coaxial to each other spaced by a base spacing and facing one another, wherein the distance sensors (8) operate in a differential system, permitting non-contact distance measurement by way of differential method, **characterized in that** on each of the brackets of the support structure (4) a measuring strip (3) is mounted, forming a measuring system consisting of two measuring strips (3) with the distance sensors (8) attached to each of these measuring strips (3) opposite to each other, differentially connected to the analyzer (5) permitting measurement of the width h1 of the conveyor belt (1), wherein each of the measuring strips (3) contains at least 3 distance sensors (8), and preferably more distance sensors (8), which distance sensors (8) are mutually paired with each other respectively on both sides of the conveyor belt (1) and arranged as spaced by a mutual distance h2 from each other, which the distance h2 is greater than the distance h1, moreover, on each bracket of the support structure (4) are located bars-observers (10), in the form of magnetometer strips (10) which are located horizontally, transversely over a surface of the conveyor belt (1) above its edge areas and each equipped with at least one magnetometer sensor (17) or more, wherein the magnetometer sensors (17) are connected by signal transmitting connection to the analyzer (5).

2. The device according to claim 1, **characterized in that**, from 3 to 20 distance sensors (8) are arranged on each of the measuring strips (3).

3. The device according to claims 1 - 2, **characterized in that** the both measuring strips (3) are so mutually located on the support structure (4) on both sides of the conveyor belt (1) that each pair of corresponding, cooperating together distance sensors (8) are paired with each other and respective distance sensors (8) are arranged in pairs in mutually matching positions opposite to each other on each of the measuring strips (3), defining the field of view (9) of the sensors.

4. The device according to claims 1 - 3, **characterized in that** the measuring strips (3) with the distance sensors (8) arranged thereon are movably positioned with ability to be moved on the brackets of the support structure (4), and their position relative to each other is such correlated, so that the distance sensors (8) in one measuring strip (3) are arranged in close synchronization with the corresponding given distance sensors (8) on the opposite, second measuring strip (3), as mutually paired with each other, wherein the displacement of the distance sensors (8) with respect to each other is performed relative to a certain reference point, preferably relative to the brackets of the support structure (4).

5. The device according to claims 1 - 4, **characterized in that** the distance h2 between the measuring strips (3) having distance sensors (8) is larger than the width h1 of the conveyor belt (1) by an amount at least twice as large as the maximum expected lateral displacement of the conveyor belt (1) on the rollers (7) during operation.

6. The device according to claims 1 - 5, **characterized in that** the measuring strip (3) with distance sensors (8) is arranged at a distance H1 from the geometric center of the cross-section of the nearest outermost edge steel cord (11) of the conveyor belt (1) and at a distance x1 from the nearest edge of the conveyor belt (1), while the distance between the edge of the conveyor belt (1) and the geometric center of the outermost edge steel cord (11) located closest to it is $b_{nom}$, wherein the distance H1 is equal to the sum of the distances $x1 + b_{nom}$ for the defect-free conveyor belt (1) having a nominal width of h1, whereas the distance H1 is smaller than the sum of the distances $x1 + b_{nom}$ at the place in which the defect (2) occurs in the edge area of the conveyor belt (1).

7. The device according to claims 1 - 6, **characterized in that** it comprises an arrangement of two bars-observers in the form of magnetometer strips (10) equipped with magnetometer sensors (17) allowing observation of the outermost edge steel cords (11), which are parts of the steel core of the conveyor belt (1), which strips are located horizontally, transverse to the direction of movement of the conveyor belt (1), on its both sides and project over its surface, whereby the magnetometer sensors (17) are located above the surface of the conveyor belt (1) and are connected by way of signal transmitting connection to the analyzer (5) in such a manner as to determine the distance H1 between the measuring strips (3) and the geometric center of the cross-section of the given outermost edge steel cord (11) located closest to the respective edge of the conveyor belt (1) and to determine the width H of the steel core of the conveyor belt (1), defined between the geometric centers of the cross-sections of the two outermost edge steel cords (11) on both opposite sides of the belt (1).

8. The device according to claims 1 - 7, **characterized in that** the length of each magnetometric strip (10) is at least equal to the sum of the distances $x1 + b_{nom}$ or greater.

9. The device according to claims 1 - 8, **characterized in that** on each magnetometer strip (10) there is a number of magnetometer sensors (17) covering the entire length of the strip in a horizontal direction, transverse to the direction of movement of the conveyor belt (1), allowing observation of the section of this conveyor belt (1), defined at least from its edge to the geometric center of the cross-section of the respective outermost edge steel cord (1) of the conveyor belt (1).

10. A method for measuring defects in edge areas of a conveyor belt (10), especially defects of the rubber carcass of edges of a conveyor belt (10) equipped with steel cords (11) forming a steel core of the belt, by detecting and determining the location of the defect in the edges of the conveyor belt (10), wherein a computer analysis is carried out using an analyzer (5) and an industrial computer (6) of a device for measuring defects in the edge areas of a conveyor belt (1) according to claim 1, the analyzer (5) connected by signal transmitting connection to distance sensors (8) located on both lateral sides of the conveyor belt (10), opposite to each other, **characterized in that**, computer analysis of signals from a number of the distance sensors (8) arranged in a paired differential arrangement with respect to each other on opposite measuring strips (3), which are built on both lateral sides of the conveyor belt (1), as well as signals from magnetometer sensors (17) connected by signal transmitting connection to the analyzer (5) and arranged on magnetometer strips (10) which are located horizontally, transversely over a surface of the conveyor belt (1) above its edge areas is carried out, wherein the distances H1, x1 and $b_{nom}$ are determined and it is checked whether the inequality 1) **H1 - x1 < $b_{nom}$** is met, where H1 - is the distance between the measuring strip (3) equipped with the distance sensors (8) arranged on it and the geometric center of the cross-sectional area of the outermost edge steel cord (11) located closest to the respective edge of the belt (1); x1 - is the distance of the measuring strip (3) equipped with the distance sensors (8) from the edge of the conveyor belt (1), and $b_{nom}$ - is the nominal width of the belt (1) edge, wherein if this inequality is met, it is considered that a defect (2) in the edge of the conveyor belt (1) at the given location is detected.

11. The method according to claim 10, **characterized in that**, during the identification of the defect (2) an analysis of the inequality $H1 - x1 < b_{nom}$ is carried out, where $b_{nom}$ is the nominal width of the belt (1) edge defined as the distance of the edge of the conveyor belt (1) from the geometric center of the cross-section of the outermost edge steel cord (11) of the steel core of the belt (1) located closest to the respective given edge of the conveyor belt (1), and if this inequity is fulfilled it is read as the occurrence of a defect (2) in the belt edge, and the location of the defect (2) is determined by way of analysis and on the basis of the signal obtained from an encoder (18) connected by way of signal transmitting connection to the analyzer (5).

**Patentansprüche**

1. Vorrichtung zur Messung von Defekten in Randbereichen eines Förderbandes (1), insbesondere von Defekten in einer Gummikarkasse von Rändern des Förderbandes (1), die einen Stahlkern des Bandes bildenden, auf Rollen (7) umlaufende Stahlseilen (11) aufweist, welche Vorrichtung eine auf beiden Seiten des Förderbandes (1) angeordnete Stützstruktur (4) umfasst, auf welcher in Untertrum des Förderbandes senkrecht zur Bewegungsrichtung des Förderbandes Abstandssensoren (8) angeordnet sind, die Signale erzeugen, die kontinuierlich an einen mit ihnen signalverbundenen elektronischen Analysator (5) übertragen und erfasst werden, wobei der Analysator (5) mit einem Industrie-Computer (6) verbunden ist, und die Stützstruktur (4) der Vorrichtung zwei neben dem Förderband (1) und einander gegenüberliegend auf beiden Seiten dieses Förderbandes (1) angeordnete Halterungen umfasst, auf denen die Abstandssensoren (8) jeweils in einem Basisabstand voneinander beabstandet, koaxial und einander zugewandt angeordnet sind, die in einer eine berührungslose Abstandsmessung mittels Differenzverfahren ermöglichenden Differenzanordnung arbeiten, **dadurch gekennzeichnet, dass** auf jeder Halterung der Stützstruktur (4) eine Messleiste (3) befestigt ist, welche Messleisten ein Messsystem bilden, das zwei Messleisten (3) mit den daran einander gegenüberliegend angebrachten und differentiell mit dem zur Messung der Breite h1 des Förderbands (1) ausgebildeten Analysator (5) verbundenen Abstandssensoren (8) umfasst, wobei jede der Messleisten (3) mindestens 3 Abstandssensoren (8) und vorzugsweise mehr Abstandssensoren (8) umfasst, die jeweils auf beiden Seiten des Förderbands (1) korrespondierend miteinander gepaart und in einem gegenseitigen Abstand h2 voneinander angeordnet sind, wobei der Abstand h2 größer als der Abstand h1 ist, und wobei ferner auf jeder Halterung der Stützstruktur (4) Balkenbeobachter (10) in Form von horizontal und quer oberhalb der Oberfläche des Förderbands (1) über seinen Randbereichen angeordneten und jeweils mit mindestens einem oder mehreren magnetometrischen Sensoren (17) versehenen magnetometrischen Leisten (10) befestigt sind, wobei die magnetometrischen Sensoren (17) mit dem Analysator (5) signalverbunden sind.

2. Vorrichtung nach Anspruch 1, **dadurch gekennzeichnet, dass** auf jeder der Messleisten (3) von 3 bis 20 Abstandssensoren (8) angeordnet sind.

3. Vorrichtung nach den Ansprüchen 1 - 2, **dadurch gekennzeichnet, dass** die beiden Messleisten (3) auf der Stützstruktur (4) auf beiden Seiten des Förderbandes (1) derart relativ zueinander angeordnet sind, dass die korrespondierenden zusammenwirkenden Abstandssensoren (8) jedes Paares miteinander gepaart und in aneinander angepassten Positionen gegenüberliegend auf jeder der Messleisten (3) angeordnet sind, so dass ein Sichtfeld (9) der Sensoren definiert ist.

4. Vorrichtung nach den Ansprüchen 1 - 3, **dadurch gekennzeichnet, dass** die Messleisten (3) mit den daran angeordneten Abstandssensoren (8) auf den Halterungen der Stützstruktur (4) bewegbar angeordnet sind und ihre relative Lage zueinander derart korreliert ist, dass die Abstandssensoren (8) in einer Messleiste (3) mit den korrrespondierenden jeweiligen Abstandssensoren (8) auf der jeweils gegenüberliegenden, anderen Messleiste (3) miteinander gepaart in enger Synchronisation angeordnet sind, wobei die relative Bewegung der Abstandssensoren (8) zueinander in Bezug auf einen bestimmten Referenzpunkt, vorzugsweise in Bezug auf die Halterungen der Stützstruktur (4), erfolgt.

5. Vorrichtung nach den Ansprüchen 1 - 4, **dadurch gekennzeichnet, dass** der Abstand h2 zwischen den Messleisten (3) mit den Abstandssensoren (8) gegenüber der Breite h1 des Förderbandes (1) um einen Betrag größer ist, der mindestens zweifach größer ist als die maximal zu erwartende seitliche Bewegung des Förderbandes (1) auf den Rollen (7) im Betrieb.

6. Vorrichtung nach den Ansprüchen 1 - 5, **dadurch gekennzeichnet, dass** die Messleiste (3) mit den Abstandssensoren (8) in einem Abstand H1 zur geometrischen Mitte des Querschnitts des nächstgelegenen äußersten randseitigen Stahlseils (11) des Förderbands (1) und in einem Abstand x1 zum nächstgelegenen Rand des Förderbands (1) angeordnet ist, während der Abstand zwischen dem Rand des Förderbands (1) und der geometrischen Mitte des ihm nächstgelegenen äußersten randseitigen Stahlseils (11) $b_{nom}$ beträgt, wobei der Abstand H1 gleich der Summe der Abstände $x1 + b_{nom}$ für das defektfreie Förderband (1) mit einer Nennbreite h1 ist, und der Abstand H1 kleiner als die Summe der Abstände $x1 + b_{nom}$ an der Stelle, an der der Defekt (2) im Randbereich des Förderbandes (1) auftritt, ist.

7. Vorrichtung nach den Ansprüchen 1 - 6, **dadurch gekennzeichnet, dass die** Vorrichtung eine Anordnung aus zwei Balkenbeobachtern in Form von den magnetometrischen Leisten (10) mit den die Beobachtung der äußersten randseitigen Stahlseile (11) des Stahlkerns des Förderbandes (1) ermöglichenden magnetometrischen Sensoren (17) umfasst, wobei die magnetometrischen Leisten (10) horizontal, quer zur Bewegungsrichtung des Förderbandes (1) auf seinen beiden Seiten angeordnet sind und über seine Oberfläche vorstehen, wobei die magnetometrischen Sensoren (17) oberhalb der Oberfläche des Förderbandes (1) angeordnet sind und mit dem Analysator (5) derart signalverbunden sind, dass der Abstand H1 zwischen der Messleiste (3) und der geometrischen Mitte des Querschnitts des jeweiligen äußersten randseitigen Stahlseils (11), der nächstgelegen zum jeweiligen Rand des Förderbandes (1) ist, und die Breite H des Stahlkerns des Förderbandes (1) zwischen den geometrischen Mitten der Querschnitte der beiden äußersten Stahlseile (11) auf beiden gegenüberliegenden Seiten des Förderbandes (1), bestimmt werden können.

8.  Vorrichtung nach den Ansprüchen 1 - 7, **dadurch gekennzeichnet, dass** die Länge jeder magnetometrischen Leiste (10) jeweils mindestens gleich der Summe der Abstände x1 + $b_{nom}$ oder größer ist.

9.  Vorrichtung nach den Ansprüchen 1 - 8, **dadurch gekennzeichnet, dass** auf jeder magnetometrischen Leiste (10) jeweils eine Anzahl von den magnetometrischen Sensoren (17) angeordnet ist, die die gesamte Länge des Streifens in einer horizontalen Richtung quer zur Bewegungsrichtung des Förderbandes (1) überdecken, so dass die Beobachtung dieses Förderbandes (1) mindestens abschnitsweise von seinem Rand zur geometrischen Mitte des Querschnitts des jeweiligen äußersten randseitigen Stahlseils (1) des Förderbandes (1) möglich ist.

10. Verfahren zur Messung von Defekten in Randbereichen eines Förderbandes (1), insbesondere von Defekten in einer Gummikarkasse von Rändern des Förderbandes (1), die einen Stahlkern des Bandes bildenden Stahlseilen (11) aufweist, durch Erfassen und Lokalisieren eines Defektes in den Rändern des Förderbandes (1), wobei eine Computeranalyse mit einem Analysator (5) und einem Industrie-Computer (6) der Vorrichtung zur Messung von Defekten in den Randbereichen des Förderbandes (1) nach Anspruch 1 durchgeführt wird, wobei der Analysator (5) mit Abstandssensoren (8) signalverbunden ist, die auf beiden Seiten des Förderbandes (1) einander gegenüberliegend angeordnet sind, **dadurch gekennzeichnet, dass** die Computeranalyse aufgrund Signalen von den mehreren Abstandssensoren (8), die paarweise in einer Differenzanordnung zueinander auf Messleisten (3) angeordnet sind, die gegenüberliegend auf beiden Seiten des Förderbandes (1) angebracht sind, sowie Signalen von mit dem Analyzator (5) signalverbundenen magnetometrischen Sensoren (17), die auf horizontal, quer über eine Oberfläche des Förderbandes (1) oberhalb seiner Randbereiche angeordneten magnetometrischen Leisten (10) angeordnet sind, durchgeführt wird, wobei die Abstände H1, x1 und $b_{nom}$ ermittelt werden und es wird überprüft, ob die Ungleichung **1) H1** - **x1** < $b_{nom}$ vorliegt, wo H1 der Abstand zwischen den mit den daran angeordneten Abstandssensoren (8) versehenen Messleisten (3) und der geometrischen Mitte des Querschnitts des äußersten, nächstgelegenen zum jeweiligen Rand des Förderbandes (1) Randseils (11) ist; x1 der Abstand der mit den Abstandsensoren (8) versehenen Messleiste (3) vom Rand des Förderbandes (1) ist und $b_{nom}$ die Nennbreite des Randes des Förderbandes (1) ist, und bei der erfüllten Ungleichung angenommen wird, dass das Defekt (2) in dem Rand des Förderbandes (1) an der jeweiligen Stelle detektiert wird.

11. Verfahren nach Anspruch 10, **dadurch gekennzeichnet, dass** während der Identifikation des Defekts (2) eine Analyse der Ungleichung H1-x1 < $b_{nom}$ durchgeführt wird, wo $b_{nom}$ die Nennbreite des Randes des Förderbandes (1) ist, die als der Abstand des Randes des Förderbandes (1) von der geometrischen Mitte der Querschnittsfläche des äußersten, nächstgelegenen zum jeweiligen Rand des Förderbandes (1) Randseils (11) des Stahlkerns des Förderbandes (1) definiert wird, wobei die Erfüllung dieser Ungleichung als Auftreten des Defekts (2) im Rand Förderbandes (1) abgelesen wird und und der Defekt (2) mittels Analyse und aufgrund Signalen von einem mit dem Analysator (5) signalverbundenen Encoder (18) lokalisiert wird.

**Revendications**

1.  Dispositif pour mesurer les défauts dans les zones de bord d'une courroie (1) de convoyeur, principalement des défauts de la carcasse en caoutchouc des bords d'une courroie (1) de convoyeur à courroie équipée en câbles (11) en acier formant le noyau d'acier de la courroie, entraînée d'un mouvement circulaire sur des rouleaux (7), le dispositif comprenant située sur les deux côtés latéraux de la courroie (1) du convoyeur une structure en portique (4) sur laquelle perpendiculairement à la direction d'avancement de la courroie du convoyeur, dans la course inférieure du convoyeur, sont placés des capteurs (8) de distance, générant des signaux qui sont transmis de manière continue par une connexion de signaux à un analyseur électronique (5) et enregistrés, l'analyseur (5) étant connecté à un ordinateur industriel (6), la construction en portique (4) du dispositif comprenant deux supports, situés près de la courroie (1) du convoyeur, un à l'opposé de l'autre des deux côtés latéraux de la courroie (1) du convoyeur, sur chacun des deux étant disposés à l'opposé, et te façon coaxiale un par rapport à l'autre, à une distance basique et dirigées un vers l'autre les capteurs (8) de distance, fonctionnant dans un système différentiel, permettant de mesurer sans contact la distance en appliquant la méthode différentielle, **caractérisé en ce que** sur chaque support de la structure en portique (4) est fixée une règle de mesure (3), formant un système de mesure des deux règles de mesure (3) avec sur chacune de ces règles de mesure (3) fixés à l'opposé un par rapport à l'autre, les capteurs (8) de distance, raccordés de façon différentielle à l'analyseur (5) permettant de mesurer la largeur h1 de la courroie (1) du convoyeur, chaque règle de mesure (3) comprenant au moins 3 capteurs (8) de distance, avantageusement plus de capteurs (8) de distance, qui sont interconnectés respectivement des deux côtés de la courroie (1) du convoyeur et disposés à une distance réciproque h2 l'un de l'autre, cette distance h2 étant supérieure à la distance h1, en outre sur chaque support de la structure en portique (4) sont fixées des règles-observateurs (10), en forme de règles magnéto-métriques (10) qui sont situés horizontalement, transversalement sur une surface de la courroie (1) du convoyeur au-dessus de ses zones de bord et sont équipées en au moins un capteur magnéto-métrique (17) ou plus chacune, et les capteurs magnéto-métriques (17) sont connectés par signaux à l'analyseur (5).

**2.** Dispositif selon la revendication 1, **caractérisé en ce que** sur chaque règle de mesure (3) sont disposés 3 à 20 capteurs (8) de distance.

**3.** Dispositif selon les revendications 1 - 2, **caractérisé en ce que** les deux règles de mesure (3) sont réciproquement situées sur la structure en portique (4) des deux côtés de la courroie (1) du convoyeur, pour que chaque paire respective des capteurs (8) de distance coopérants soit interconnectée et disposée dans des positions réciproquement ajustées, à l'opposé l'un de l'autre sur chaque des règles de mesure (3), définissant le champ de vision (9) des capteurs.

**4.** Dispositif selon les revendications 1 - 3, **caractérisé en ce que** les règles de mesure (3) avec les capteurs (8) de distance disposés sur ces règles, sont montées mobiles, avec la possibilité de déplacement sur les supports de la structure en portique (4), et leur position une par rapport à l'autre est corrélée de façon à ce que les capteurs (8) de distance sur une règle de mesure (3) soient montés strictement synchronisés avec leurs capteurs (8) de distance respectifs sur la deuxième règle de mesure (3) opposée, soit une interconnexion réciproque, le déplacement réciproque des capteurs (8) de distance étant réalisé par rapport à un point de référence défini, avantageusement par rapport aux supports de la structure en portique (4).

**5.** Dispositif selon les revendications 1 - 4, **caractérisé en ce que** la distance h2 entre les règles de mesure (3) avec les capteurs (8) de distance est supérieure à la largeur h1 de la courroie 1 du convoyeur d'une grandeur au moins deux fois plu grande que le déplacement transversal maximum prévisible de la courroie (1) du convoyeur sur les rouleaux (7) en cours d'exploitation.

**6.** Dispositif selon les revendications 1 - 5, **caractérisé en ce que** la règle de mesure (3) avec les capteurs (8) de distance est montée à une distance H1 du centre géométrique de la section transversale du plus proche le plus à l'extérieur, câble en acier (11) de bord de la courroie (1) du convoyeur, et à une distance x1 du bord le plus proche de la courroie (1) du convoyeur, tandis que la distance entre le bord de la courroie (1) du convoyeur et le centre géométrique du plus à l'extérieur câble en acier (11) de bord situé le plus près de ce bord est $b_{nom}$, la distance H1 étant égale à la somme des distances $x1 + b_{nom}$ pour une courroie (1) de convoyeur sans défauts de largeur nominale h1, tandis que la distance H1 est inférieure à la somme des distances $x1 + b_{nom}$ à la place présentant un défaut (2) dans la zone de bord de la courroie (1) du convoyeur.

**7.** Dispositif selon les revendications 1 - 6, **caractérisé en ce qu'il** comporte un système de deux règles-observateurs en forme de règles magnéto-métriques (10) avec des capteurs magnéto-métriques (17) permettant l'observation des câbles en acier (11) de bord, les plus à l'extérieur, faisant partit du noyau en acier de la courroie (1) du convoyeur, ces règles étant montées verticalement, transversalement à la direction d'avancement de la courroie (1) du convoyeur, des deux de ses côtés et sont en saillie au dessus de sa surface, les capteurs magnéto-métriques (17) étant situés au dessus de la surface de la courroie (1) du convoyeur et connectés par signaux à l'analyseur (5), de manière à permettre de définir la distance H1 entre la règle de mesure (3) et le centre géométrique de la section transversale le plus à l'extérieur câble en acier (11) de bord donné situé le plus près du bord donné de la courroie (1) du convoyeur, et la largeur H du noyau en acier de la courroie (1) du convoyeur, définie entre les centres géométriques des sections transversales des deux les plus à l'extérieur câbles en acier (11) de bord.

**8.** Dispositif selon les revendications 1 - 7, **caractérisé en ce que** la longueur de chaque règle magnéto-métrique (10) est égale au moins à la somme des distances $x1 + b_{nom}$ ou supérieure.

**9.** Dispositif selon les revendications 1 - 8, **caractérisé en ce que** sur chaque règle magnéto-métrique (10) se trouve un nombre de capteurs magnéto-métriques (17) couvrant toute la longueur de la règle dans la direction horizontale, transversale à la direction d'avancement de la courroie (1) du convoyeur, permettant l'observation de cette courroie (1) du convoyeur au moins sur le tronçon depuis son bord vers le centre géométrique de la section transversale le plus à l'extérieur câble en acier (11) de bord donné de la courroie (1) du convoyeur.

**10.** Procédé de mesure des défauts dans les zones de bord d'une courroie (10) de convoyeur, principalement des défauts de la carcasse en caoutchouc des bords d'une courroie (10) de convoyeur à courroie équipée en câbles (11) en acier formant le noyau d'acier de la courroie, par la détection et la définition de la position du défaut apparu aux bords de la courroie (10) du convoyeur, dans lequel on mène une analyse d'ordinateur en utilisant un analyseur (5) et un ordinateur industriel (6) du dispositif pour mesurer les défauts dans les zones de bord d'une courroie (1) de convoyeur selon la revendication 1, l'analyseur (5) connecté par connexion de transmission de signaux avec les capteurs (8) de distance montés des deux côtés latéraux de la courroie (10) du convoyeur, un à l'opposé de l'autre, **caractérisé en ce que** on mène une analyse d'ordinateur des signaux d'un certain nombre des capteurs (8) de distance, montés de façon interconnectée dans un système différentiel sur les règles de mesure (3), lesquelles sont montées des deux côtés latéraux de la courroie (1) du convoyeur et des signaux des capteurs magnéto-métriques (17) connectés par connexion de transmission de signaux à l'analyseur (5) et montés sur les règles magnéto-métriques (10), lesquelles sont situées horizontalement, transversalement au dessus de la surface de la courroie (1) du convoyeur au dessus de ses zones de bord, ceci étant on définit les distances H1, x1 et $b_{nom}$ et on vérifie si l'inéquation est remplie **1) H1-x1< b nom,** où H1 - est la distance entre la règle de mesure (3) avec les capteurs (8) de distance, et le centre géométrique de la section transversale du plus à l'extérieur câble en acier (11) de bord situé le plus près du bord donné de la courroie (1); x1 - est la distance de la règle de mesure (3) avec les capteurs (8) de distance du bord de la courroie (1) du convoyeur , tandis que $b_{nom}$ - est la largeur nominale du rebord de la courroie (1), ceci étant si cette inéquation est remplie on considère qu'un défaut (2) a été détecté sur le bord de la courroie (1) du convoyeur dans une position donnée.

11. Procédé selon la revendication 10, **caractérisé en ce que** lors d'une identification de défaut (2) on procède à une analyse de l'inéquation H1-x1 < $b_{nom}$, où $b_{nom}$ est la largeur nominale du rebord de la courroie (1) définie comme étant la distance du bord de la courroie (1) du convoyeur du centre géométrique de la section transversale du plus à l'extérieur câble en acier (11) de bord du noyau en acier de la courroie (1), situé le plus près du bord de la courroie (1) du convoyeur, et cette relation remplie est interprétée comme la présence d'un défaut (2) dans un bord de la courroie et on définit la position du défaut (2) présent par l'analyse et selon le signal reçu depuis un encodeur (18) connecté par connexion de transmission de signaux avec l'analyseur (5).

Fig. 1

Fig.2

Fig. 2a

**EP 4 534 986 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1811266 A
- JP 20002277384 A
- US 4031741 A
- WO 2009061731 A1
- PL 215143
- AU 2020203474
- AU 2012216769 A1
- US 2023091198 A1